# EUROPEAN PATENT APPLICATION

(11) **EP 1 081 232 A1**
(43) Date of publication of application: **07.03.2001**
(21) Application number: 99202805.0
(22) Date of filing: 30.08.1999
(51) Int. Cl.: C12Q 1/42, C12Q 1/18, A01N 61/00, A61K 35/00

(54) **Novel target for antifungals**

(71) Applicant: K.U. LEUVEN RESEARCH & DEVELOPMENT, 3000 Leuven (BE)
(72) Inventor: Thevelein, Johan, 3001 Heverlee (BE); van Dijck, Patrick, 3271 Zichem (BE)
(74) Representative: Bird, William Edward

(57) **Abstract**

The use of an enzyme found in fungi, bacteria, insects, and nematodes as a target in a screening assay is described by means of which agents capable of inhibiting the function of that enzyme may be identified. The screening assay may include complete cell or purified-enzyme assays. In particular, the present invention relates to a screening assay for inhibitors or suppressors of sugar alcohol phosphatases or sugar phosphatases as well as preparations, in particular, pharmaceutical preparations, which include the inhibitor or suppressor obtained from the screening assay.

Inhibitors are described as well as applications in biocides and anitfungal pharmaceuticals.

## Description

The present invention relates to the use of an enzyme found in fungi, bacteria, insects, and nematodes as a target in a screening assay by means of which agents capable of inhibiting the function of that enzyme may be identified. The screening assay may include complete cell or purified-enzyme assays. In particular, the present invention relates to a screening assay for inhibitors or suppressors of sugar alcohol phosphatases or sugar phosphatases as well as preparations, in particular, pharmaceutical preparations, which include the inhibitor or suppressor obtained from the screening assay.

### TECHNICAL BACKGROUND

Fungal cells and fungal spores have an amazing capacity for adaptation to survival under stress conditions and resume their vital functions as soon as the stress condition is removed. These organisms withstand freezing, strong vacuum, high doses of ionizing radiation, high pressure, osmotic stress and extreme temperatures without suffering damage and many of them accumulate the non-reducing disaccharide trehalose as a protein and membrane protectant.

The biosynthesis of trehalose consists of two enzymatic steps catalyzed by trehalose-6-phosphate synthase (TPS, expressed by the *TPS1* gene), which synthesizes trehalose-6-phosphate and by trehalose-6-phosphate phosphatase (TPP, expressed by the *TPS2* gene) which forms trehalose. Detailed information on the composition and function of the trehalose synthase complex can be found in Reinders A. et al. (Mol. Microbiol. 24, 687-695, 1997) and Bell W. et al. (J. Biol. Chem. 276, 33311-33319, 1998). In addition to its classical role in storage sugar accumulation, trehalose metabolism is known to play an important role in stress resistance, control of glucose influx into glycolysis and glucose-induced signaling.

As described in the article by De Virgilio et al., J.Biochem., vol. 212, 1993, pages 315-323, the disruption of the *TPS2* gene in *Saccharomyces cerevisiae* causes loss of trehalose-6-phosphatase activity and accumulation of trehalose-6-phosphate whereas the wild type strain had hardly detectable levels of the trehalose-6-phosphate. It appears that accumulation of high levels of trehalose-6-phosphate is inhibitory to growth and survival, since conditions that lead to the accumulation of trehalose in wild type cells cause loss of viability of the *tps2* disruption mutant. This is observed for instance upon incubation at 37°C and when the cells are grown into stationary phase.

The incidence of fungal infections in patients has dramatically increased in the last 20 years (Klepser M. E. et al., Ann. Pharmacother., vol. 32, 1998, pages 1353 ― 1361). Especially *Candida albicans* has become an important opportunistic pathogen. Patients suffering from immunodeficiency are especially vulnerable to infections with *Candida* and other fungi. Although *Candida albicans* is a common microorganism, found in the digestive tract and cavities of 10 to 50% of normal humans, upon a decreased resistance to infection in the host or upon administration of antibiotics for a long term or upon a surgical invasion, these microorganisms proliferate abnormally, damage tissues, and can enter the blood. Treatment is often hampered by the fact that many agents which are active against fungi also are toxic to mammalian cells, leading to a low therapeutic index and undesirable side effects in the patient.

A selection of drugs is available to combat fungal infections: Amphotericin B, Flucytosine, Ketoconazole, Miconazole, Fluconazole, and Itraconazole (Polak A., Progress in Drug Research, Vol. 49, 1997, pages 219-318). All these drugs have specific drawbacks. Amphotericin B is very efficacious because of its fungicidal action but is also relatively toxic to the patient especially to the renal functions. Flucytosine has a limited antifungal spectrum and the appearance of resistant cells is very frequent. As a result it is only used in combination with other drugs. Ketoconazole has a broad spectrum and is very useful for deep mycoses but it shows significant interactions with other drugs and causes endocrinopathies and hepatopathies. It cannot be used in immunosuppressed patients. Miconazole suffers from similar problems as Ketoconazole. Fluconazole and ltraconazole are more specific, less toxic and more efficacious than Ketoconazole and Miconazole. However Fluconazole is active against fewer fungi. Itraconazole is highly lipophilic and as a result reaches only low levels in most body fluids. As a result there is a requirement for novel antifungals with broader spectrum, higher efficacity and less side-effects.

The azole class of antifungals is also widely used in agriculture to combat plant pathogenic fungi (Adams D.J., 1997, In: Molecular genetics of drug resistance, eds. Hayes J. H., Wolf C. R., Harwood Academic Publishers, The Netherlands).

A common problem with all existing drugs is the appearance of resistance in the fungal pathogens. The longer the drugs are in use the more resistance is observed. This requires the constant development of new antifungal drugs for which the pathogens still show great sensitivity.

The same problem applies to agriculture where a steady increase in resistance to antifungals is observed in many plant pathogenic fungi (Adams D. J., 1997, In Molecular genetics of drug resistance, eds. Hayes J. H., Wolf C. R., Harwood Academic Publishers , The Netherlands).

Some antifungal drug discovery efforts have been directed at components of the fungal cell or its metabolic pathways which are unique to fungi, and hence might be used as targets of new therapeutic agents. Ideally, these should act on the fungal pathogen without undue toxicity to host cells. Because no single approach is effective against all fungal pathogens and because of the possibility of developed resistance to previously effective antifungal compounds, there remains a need for new antifungal agents with novel mechanisms of action. An essential aspect of meeting this need is the selection of an appropriate component of fungal structure or metabolism as a therapeutic target.

Despite the increased use of rational drug design, a preferred method continues to be the mass screening of compound libraries for active agents by exposing cultures of pathogens to the test compounds and assaying for inhibition of growth. In testing thousands or tens of thousands of compounds, however, a correspondingly large number of fungal cultures must be grown over time periods which are relatively long. Moreover, a compound which is found to inhibit fungal growth in culture may be acting not on the desired target but on a different, less unique fungal component, with the result that the compound may act against host cells as well and thereby produce unacceptable side effects. Consequently, there is a need for assay or screening methods which more specifically identify those agents that are active against a certain intracellular target. Additionally, there is a need for assay methods having greater throughput, i.e., which reduce the time and materials needed to test each compound of interest.

It is an object of the present invention to provide a screening assay to identify pharmaceuticals with improved effectivity against fungal disease.

It is an object to provide a method of identifying useful antifungal drugs and coagents for antifungal drugs, which reduce the side effects of conventional drugs.

### SUMMARY OF THE INVENTION

The present invention includes a novel target for antifungal agents, such as antifungal drugs or fungicides, that is not required for growth of the fungus under standard conditions in vitro but that is specifically required for survival of the fungus under all conditions of reduced growth or absence of growth and all other conditions which in some way deviate from optimal growth conditions and/or apply stress to the fungus.

In accordance with embodiments of the present invention trehalose-6-phosphate phosphatase and similar phosphatases converting sugar-, glycerol- and sugar alcohol phosphates to the corresponding unphosphorylated compounds as well as the genes expressing these enzymes are targets for antifungal agents, such as antifungal drugs or fungicides, either alone or in combination with agents, e.g. drugs, inhibiting growth of the cells. Inhibition of the enzyme in fungal pathogens, either directly or by blocking its expression from the corresponding gene, leads to accumulation of trehalose-6-phosphate rather than trehalose, the cells will enter into a vicious circle of stress and enhanced synthesis of trehalose-6-phosphate as a reaction against the stress, and they will rapidly die. Preferably, other parallel pathways to produce trehalose are inhibited so that the targeted cell has high trehalose phosphate levels and low trehalose levels thus doubly weakening the cell against attack by antifungal agents such as antifungal drugs or fungicides or by the immune system of the host.

The present invention includes inhibitors of trehalose-6-phosphate phosphatase. Inhibition of this enzyme leads to much faster elimination of the fungal pathogen compared to inhibition of targets which are merely essential for growth and therefore only fungistatic rather than fungicidal.

The present invention is also applicable to other organisms that synthesize large quantities of trehalose, using this sequence of enzyme reactions, such as bacteria, insects and nematodes.

Non-optimal growth conditions generally prevail during growth of fungal pathogens in host organisms and under these conditions the target therefore is essential. Trehalose-6-phosphate phosphatase, the second enzyme in the biosynthesis pathway of trehalose, converts trehalose-6-phosphate into trehalose. All fungi contain trehalose, which serves as a storage carbohydrate and as a stress protectant. Because of these functions trehalose is accumulated under unfavorable growth conditions and in survival forms where it can reach very high concentrations. Trehalose-6-phosphate on the other hand is normally only found in very low concentrations and its accumulation at high levels is toxic to the cells. It is synthesized by trehalose-6-phosphate synthase, which is encoded by the *TPS1* gene. Inactivation of the *TPS2* gene, which encodes trehalose-6-phosphate phosphatase, renders fungal cells hypersensitive to stress conditions, for example, commonly used antifungals specifically under non-optimal growth conditions. As an example this is demonstrated for the fungus *Saccharomyces cerevisiae*. The genes of trehalose metabolism, including the *TPS2* gene, are also present in *Candida albicans*, an important human pathogen. Equivalent genes to, or names for *TPS2* are *HOG2*, *PFK3*, *D4416*, *YD8554.07*, *YDR074W*. In addition, all existing knowledge indicates that trehalose metabolism uses the same enzymes, trehalose-6-phosphate synthase and phosphatase, in all fungi, including fungal pathogens of humans, mammals and other animals, and plants. Under non-optimal growth conditions and a variety of stress conditions fungal cells, including those of pathogens such as *Candida albicans*, accumulate large quantities of trehalose and often also other sugars or polyols. Inhibitors of trehalose-6-phosphate phosphatase cause accumulation of trehalose-6-phosphate. Mutants deficient in the trehalose-6-phosphate phosphatase enzyme or cells treated with the novel inhibitors in accordance with the present invention accumulate large quantities of trehalose-6-phosphate under these conditions, which is highly toxic to the cells because it is a strong acid. It acts as a pleiotropic inhibitor of a wide range of essential cellular components and cellular defense systems. Because the accumulation of trehalose-6-phosphate itself is a stress condition to the cell and because the trehalose-6-phosphate is synthesized as a reaction to the stress condition, the cells enter into a vicious circle after which they finally die.

The present invention also includes all similar metabolic situations, such as the conversion of glycerol-3-phosphate to glycerol, mannitol-1-phosphate to mannitol, sorbitol-6-phosphate to sorbitol, arabitol-5-phosphate to arabitol, erythritol-4-phosphate to erythritol. Glycerol is generally accumulated in fungi under osmotic stress. Depending on the species sugar alcohols are accumulated together with trehalose. This is probably the case for instance in *Aspergillus fumigatus*.

It should be emphasized that a purpose of the antifungal agents in accordance with the present invention is not only to block the growth of the fungus but preferably to eradicate the fungus or to assist in its eradication. Hence, although it is useful to have an antifungal that blocks the growth of the fungus, it is much better to have an antifungal that kills the fungus. Especially in immunocompromised hosts the difference between mere inhibition of growth of the fungus and actual killing of the fungus is important since these patients will have a reduced capacity to eliminate the fungus themselves when its growth is merely inhibited.

The synthesis of trehalose is induced in fungi under a variety of stress conditions. In yeast it is part of the general stress response mechanism, which is mediated by STRE-elements in the promoter of genes involved in stress protection (e.g. heat shock proteins, catalase, *TPS1* encoding trehalose-6-phosphate synthase, etc.). Hence, if the growth of the fungus is inhibited by antifungals it may also initiate the stress response. This means that inhibitors that act on the trehalose-6-phosphate phosphatase lock the fungus into a vicious circle. Because it is stressed it reacts with the stress response mechanism of which stimulation of trehalose synthesis forms part. However, because the phosphatase is inhibited, trehalose-6-phosphate will be accumulated instead of trehalose and it will become even more stressed, inducing an even stronger stress response resulting in more toxic trehalose-6-phosphate, and so on.

The *tps2* mutant in *Saccharomyces cerevisiae* is not only temperature sensitive but also osmosensitive. It has been isolated as an osmosensitive mutant and the gene called *HOG2*. In tissues of organisms, water availability is usually restricted and the fungal pathogens are therefore generally osmostressed. Since inhibition of trehalose-6-phosphate phosphatase renders the cells osmosensitive, the fungus will also be unable to survive because of this reason.

The present invention includes all antifungal agents, e.g. antifungal drugs or fungicides, that inhibit enzymes converting with a low or high degree of specificity, sugar phosphates into sugars or sugar alcohol phosphates into sugar alcohols that are accumulated in large quantities for instance, but not exclusively, under conditions deviating from the optimal growth condition or as a reaction to stress conditions.

The present invention also includes all biocides acting on insects, nematodes, bacteria and other organisms accumulating large quantities of trehalose and/or similar stress-protective sugars or sugar alcohols and inhibiting enzymes converting with a low or high degree of specificity sugar phosphates into sugars or sugar alcohol phosphates into sugar alcohols that are accumulated in large quantities for instance, but not exclusively, under conditions deviating from the optimal growth condition or as a reaction to stress conditions.

The present invention includes a screening assay for identifying inhibitors which inhibit a first cell enzyme converting with a low or high degree of specificity a sugar phosphate into a sugar or a sugar alcohol phosphate into a sugar alcohol that are accumulated in large quantities by cells for instance, but not exclusively, under conditions deviating from the optimal growth condition or as a reaction to stress conditions, the inhibition being either directly of the enzyme or indirectly, e.g. by suppressing the expression of the corresponding gene; the method comprising the steps of
Step 1: contacting a candidate inhibitor with a biological medium comprising the sugar phosphate or sugar alcohol phosphate and the first enzyme;
Step 2: measuring activity which depends upon the activity of the first enzyme;
Step 3: repeating steps one and two with further candidate inhibitors; and
Step 4: selecting those candidate inhibitors which reduce activity of the enzyme compared with the same medium without the inhibitor under the same conditions.

The first enzyme is preferably a phosphatase which synthesizes a sugar or sugar alcohol as a reaction to stress. The reduction in activity is preferably at least 25%, more preferably at least 50%, more preferably at least 75%, more preferably at least 85% and most preferably at least 95%. In a separate assay, the activity of the second enzyme which is involved in the synthesis of the corresponding sugar phosphate or sugar alcohol phosphate is assessed and the selecting step preferentially involves selection of inhibitors which reduce the activity of the first enzyme while maintaining a viable activity of the second enzyme Viable activities are considered to be at least 25%, more preferably at least 50% and most preferably at least 75% of the activity of the second enzyme in the same medium under the same conditions but without the inhibitor.

The medium may include a pure enzyme or pure enzymes, sub-cellular organelles or sub-cellular non-organelle components (*in vitro* screening), a cell culture, or animal tissue, plant tissue or a plant or an animal (*in vivo* screening). The sub-cellular organelles or sub-cellular non-organelle components or the cell culture may be obtained from the target organism, e g cells from insects or nematodes or cells of fungi or bacteria, but the present invention is not limited thereto.

The first enzyme may be one or more of trehalose-6-phosphatase, glycerol-3-phosphatase, mannitol-1-phosphatase, sorbitol-6-phosphatase, arabitol-5-phosphatase, or erythritol-4-phosphatase or any similar enzyme controlling a metabolic pathway which has an intermediary compound which is normally produced as a reaction to stress conditions and/or is toxic to the cell in high concentrations.

The present invention may provide a screening assay for an inhibitor of the first enzyme in fungi. Yeast cells are extracted by vortexing in the presence of glass beads. After clearing of the extract by low-speed centrifugation, it is desalted on a gel filtration column, i. e. Sephadex G25. The final cell extract is used to measure the activity of the first enzyme. To screen the inhibitors, different concentrations of candidate compounds are added and the residual activity of the first enzyme is measured. For example, when the first enzyme is TPP, the substrate, trehalose-6-phosphate, is added to the final cell extract and either the formation of trehalose or free phosphate is then measured. Inhibitors are selected based on their ability to inhibit TPP. Ideally, inhibitors are selected which inhibit TPP but not TPS.

Filamentous fungi may be extracted after freezing in liquid nitrogen. The frozen mycelia are extracted by grinding in a mortar. After addition ofthe buffer, the cell extracts are cleared by low-speed centrifugation and desalted over a Sephadex column. To screen for inhibitors, different concentrations of these compounds are added and the residual activity of the first enzyme is measured. When the first enzyme is TPP, trehalose-6-phosphate is added to the cell extracts and either the phosphate or the trehalose that is generated is measured. Inhibitors are selected based on their ability to inhibit TPP. Ideally, inhibitors are selected which inhibit TPP but not TPS.

The present invention may provide a screening assay for an inhibitor of the first enzyme in nematodes. Nematodes are extracted after freezing in liquid nitrogen. The frozen nematodes are extracted by grinding in a mortar. After addition of the buffer, the cells are cleared by low-speed centrifugation and desalted over a Sephadex column. To screen for inhibitors, different concentrations of candidate compounds are added and the residual activity of the first enzyme is measured. When the first enzyme is TPP, trehalose-6-phosphate is added to the cell extract and either the phosphate or the trehalose that is generated is measured. Inhibitors are selected based on their ability to inhibit TPP. Ideally, inhibitors are selected which inhibit TPP but not TPS.

The present invention may provide a screening assay for an inhibitor of the first enzyme in insects. Insects or parts of the insects are extracted after freezing in liquid nitrogen. The frozen insects are extracted by grinding in a mortar. After addition of the buffer, the cell extracts are cleared by low-speed centrifugation and desalted over a Sephadex column. To screen for inhibitors, different concentrations of candidate compounds are added to the cell extract and the residual activity of the first enzyme is measured. When the first enzyme is TPP, trehalose-6-phosphate is added to the cell extract and either the phosphate or the trehalose that is generated is measured. Inhibitors are selected based on their ability to inhibit TPP. Ideally, inhibitors are selected which inhibit TPP but not TPS.

The present invention may provide a screening assay for an inhibitor of a first enzyme in bacteria. Bacterial cells are extracted by vortexing in the presence of glass beads. After clearing of the extract by low-speed centrifugation, it is desalted on a gel filtration column, i. e. Sephadex G25. The final cell extract is used to measure the TPP activity To screen the inhibitors, different concentrations of candidate compounds are added and the residual activity of the first enzyme is measured. When the first enzyme is TPP the substrate, trehalose-6-phosphate, is added to the cell extract and either the formation of trehalose or free phosphate is then measured. Inhibitors are selected based on their ability to inhibit TPP. Ideally, inhibitors are selected which inhibit TPP but not TPS

According to the method for the identification of enzyme inhibitors of the present invention, assays may be carried out both in whole-cell preparations or in *ex vivo* cell-free systems. In each instance, the assay target is an enzyme converting with a low or high degree of specificity a sugar phosphate into a sugar or a sugar alcohol phosphate into a sugar alcohol that are accumulated in large quantities by cells for instance, but not exclusively, under conditions deviating from the optimal growth condition or as a reaction to stress conditions, the inhibition of which enzyme significantly attenuates cell growth or is lethal. Test compounds which are found to inhibit a target enzyme in an assay of the present invention are thus identified as potential pharmaceutically or biologically active agents. It is expected that the assay methods of the present invention will be suitable for both small- and large-scale screening of test compounds, as well as in quantitative assays such as serial dilution studies wherein the target enzyme is exposed to a range of test compound concentrations.

When the method of the present invention is carried out as a whole-cell assay, the target enzyme is an intracellular enzyme and the entire, living cells are exposed to the test compound under culture conditions in which the target enzyme is produced, e.g. during non-optimal growth, stress situations. Such conditions, including essential nutrients, optimal temperatures and other parameters, depend upon the particular fungal, bacterial, insect, nematode strain being targeted. The step of determining inhibition of the enzyme (step 2 above) may be carried out by observing the cell culture's growth or lack thereof; such observation may be made visually, by optical densitometric or other light absorption/scattering means, or by other suitable means, whether manual or automated.

In the above whole-cell assay, an observed lack of cell growth may be due to inhibition of the target enzyme or an entirely different effect of the test compound, and further evaluation is required to establish the mechanism of action and to determine whether the test compound is a specific inhibitor of the target enzyme. Accordingly, and in a preferred embodiment of the present invention, the method may be performed as a paired-cell assay in which each test compound is separately tested against two different sets of cells, the first having a lower enzyme activity than that of the second and thereby being more susceptible to inhibition of the enzyme.

Compounds which are found to inhibit the first, more susceptible cells but not the second are likely to have acted specifically on the target enzyme and not via a different mechanism.

One manner of achieving differential susceptibility is by using a first cell which has diminished enzyme activity relative to that of a wild-type cell, as for example a mutant strain

Alternatively, or in conjunction with the above, differential susceptibility to target enzyme inhibitors may be obtained by using a second fungal cell which has increased enzyme activity relative to that of a wild-type cell, as for example one which has been genetically manipulated to cause overexpression of the enzyme. Such overexpression can be achieved by placing into a wild-type cell a plasmid carrying the gene for the target enzyme.

Preferred is a method in which the differentiated target enzyme activity is produced by subjecting one set of cells to a stress or non-optimal growth situation which favors enzyme activity and a second control set without the stress or non-optimal growth promoter.

The present invention also includes any inhibitor found by any of the above screening assays. In particular, the present invention includes any inhibitor found by any of the above screening assays used in a pharmaceutical preparation either alone or in combination with an antifungal drug. The present invention also includes any inhibitor found by any of the above screening assays used in a biocide acting on insects, nematodes, bacteria or other organisms accumulating large quantities of a sugar alcohol or a sugar in response to stress.

The present invention also includes a screening assay for identifying inhibitors which inhibit a first cell enzyme converting with a low or high degree of specificity a sugar phosphate into a sugar or a sugar alcohol phosphate into a sugar alcohol that are accumulated in large quantities by cells for instance, but not exclusively, under conditions deviating from the optimal growth condition or as a reaction to stress conditions, the inhibition being either directly of the enzyme or indirectly, e.g. by suppressing the expression of the corresponding gene; the method comprising the steps of:
Step 1: contacting a candidate inhibitor with a biological medium comprising the sugar phosphate or sugar alcohol phosphate and the first enzyme;
Step 2: measuring activity which depends upon the activity of the first enzyme; Step 3: repeating steps one and two with further candidate inhibitors;
Step 4: selecting those candidate inhibitors which reduce activity of the enzyme compared with the same medium without the inhibitor under the same conditions.
Step: 5 contacting the selected candidate inhibitors with a biological medium comprising whole cells having the first enzyme as an intracellular enzyme; and
Step 6: selecting those candidate inhibitors which reduce the growth of the cells.

### BRIEF DESCRIPTION OF THE DRAWINGS

Figure 1 gives a representation of the trehalose biosynthesis in *Saccharomyces cerevisiae* as a two step process.

Figures 2A and B show the growth curves of wild type and prototrophic *tps2*Δ strains in the presence of different concentrations of Itraconazole at 37°C, respectively.

Figure 3 shows the growth curves of prototrophic *tps2*Δ and wild type strains in the presence of 10⁻⁷ M of Itraconazole at 33°C.

Figure 4 shows the growth curves of prototrophic *tps2*Δ (PVD23) and wild type (PVD32) strains in the presence of 10⁻⁶ M of Ketoconazole at 33°C.

Figures 5A and B show the growth curves of diploid prototrophic wild type (PVD190) and diploid heterozygous *tps2*Δ (PVD191) strains in the presence of different concentrations of Itraconazole at 33°C.

Figure 6 shows the effect of Itraconazole on the growth of wild type (PVD32) and *tps2*Δ (PVD23) strains on YPD plates.

Figure 7 shows the effect of osmotic and heat stress on the growth of wild type (PVD32) and *tps2*Δ (PVD23) strains on YPD plates.

Figures 8 and 9 show the inhibition of trehalose-6-phosphate activity in strain PVD45 by NEM and DTNB.

### DESCRIPTION OF THE ILLUSTRATIVE EMBODIMENTS

The present invention will be mainly described with reference to trehalose-6-phosphate and yeast cells but the present invention is not limited thereto but only by the claims. In particular the present invention may find advantageous use in the control, including plant, animal or human therapeutic control of fungi, nematodes, bacteria and insects.

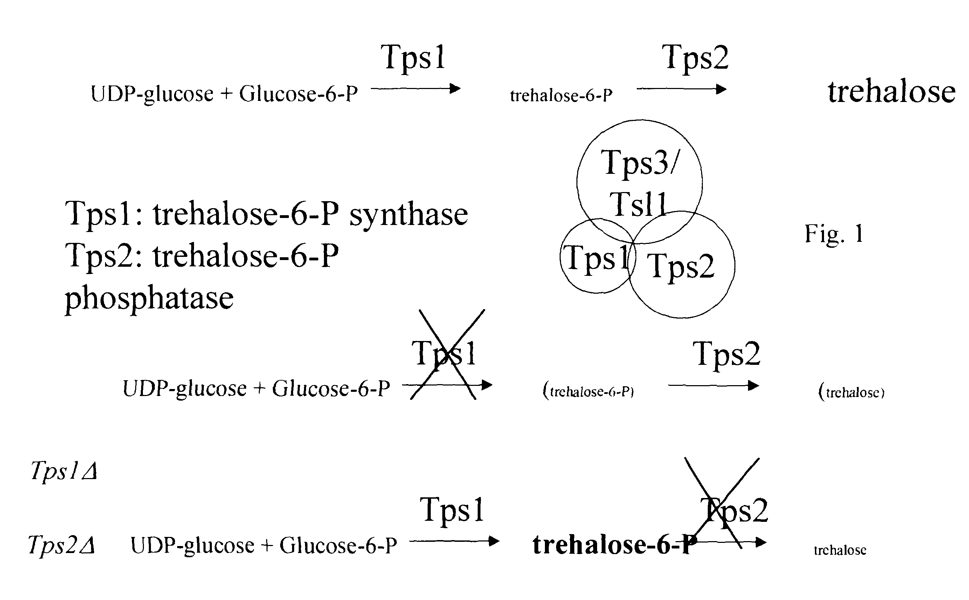

Fig. 1 is a schematic representation of the metabolic pathway within yeasts, other fungi, bacteria, nematodes and insects producing trehalose. Trehalose is synthesized from glucose-6-phosphate and UDP-glucose, catalyzed by trehalose-6-phosphate synthase (TPS), which is encoded by the gene *TPS1* in yeast, to form trehalose-6-phosphate which is further processed to trehalose by trehalose-6-phosphatase (TPP) which is encoded by the gene *TPS2* in yeasts. Further, additional genes *TPS3* and *Tsl1* are believed to encode proteins which only play a regulatory role.

The promoters of the *TPS1* and *TPS2* genes are highly stress dependent and TPS is very active under bad growth conditions such as during growth on non-fermentable carbon sources, during nutrient limitation, e.g. during the stationary phase and during growth at high temperatures. Inhibition of the TPP enzyme or mutation or elimination of the *TPS2* gene while leaving the TPS enzyme intact, results in an increase in the trehalose-6-phosphate levels in the cell under conditions where wild type cells accumulate trehalose. The present inventors have been the first to determine that specific inhibition of the TPS enzyme while maintaining the viability of the TPS enzyme makes the cell more prone to attack by antifungal agents. That is, the amount of antifungal agent required to stop growth or to kill the cell is reduced. This is particularly advantageous as the commonly used antifungal agents have serious side effects and any method of reducing the concentrations having a significant therapeutic effect is valuable. The trehalose metabolic pathway does not play an important role in humans and other mammals, so that a specific inhibitor to TPP, offers the possibility of reduced, few or no side effects The present invention therefore sets as one of its objects the development of a specific inhibitor for TPP. Accordingly, embodiments of the present invention relate to a screening assay for detecting specific inhibitors or TPP.

### MATERIALS AND METHODS

### METHOD FOR MEASUREMENT OF TREHALOSE -6-PHOSPHATASE ACTIVITY

Methods for the measurement oftrehalose-6-phosphatase activity may comprise methods for the measurement of the trehalose that is released from trehalose-6-phosphate and/or the measurement of the phosphate that is released from the trehalose-6-phosphate.

### 1. Preparation of the extracts:

a) cultures are grown to the desired density and are cooled quickly on ice;
b) preferably, at least 400 mg of cells are harvested by centrifugation for 3 min at 3000 rpm;
c) cells are washed twice with ice-cold distilled water;
d) cells are resuspended in 990 µl extraction buffer consisting of 968 µl of 50 mM Imidazole (Merck 1.04716 (RT)), 2 µl of 0.5 M EDTA (Tritriplex III Merck 1 08418 (RT)), 20 µl of 0.1 M MgCl₂ (UCB 94046076 (RT),
d) cells are transferred to screw capped tubes and an equivalent of 0.5 ml glass beads with 0.5 mm diameter are added;
e) just before extraction 10 µl of a 100 mM PMSF solution (Sigma P7626 (RT)) in methanol is added to the cell mixture ;
d) the extraction is performed in a Fastprep apparatus (BIO101) for 20 seconds at level 4, and is repeated three times with a cooling step on ice in between. Alternatively the extraction can be performed on a vortex apparatus in glass tubes,
e) extracts are centrifuged at 4°C for 20 min at 14000 rpm;
f) 20 µl of the supernatant is loaded on a small Sephadex G25 column, made in a blue tip containing a siliconized glass bead. The tip is filled completely with pre-equilibrated G25 Sephadex (50 mM Tricine (Sigma T-0377 (RT)) buffer (pH7). The tips are centrifuged once before application of the extract for 1 min at 800 rpm The extract is centrifuged for 1 min at 1000 rpm;
g) 200 µl of the eluate is added to 140 µl Assay I solution consisting of 40 µl of 200 mM Tricine buffer (pH7), 20 µl of 0.1 M MgCl₂, 20 µl of 25 mM trehalose-6-phosphate (Sigma) and 60 µl H₂O. For the Assay I control mixture 20 µl trehalose-6-phosphate is omitted and replaced by 20 µl H₂O.
h) the assay mixture and the control mixture are incubated for 30 min at 30°C;
i) the assay mixture and the control mixture are boiled for 5 min and cooled down to room temperature.
j) centrifugation of the micro-centrifuge tubes for 5 min at 14000 rpm

### 2. Measurement of trehalose that is released from trehalose-6-phosphate.

One method of calculating the amount of trehalose released from trehalose-6-phosphate is by performing two steps: the hydrolysis of trehalose by trehalase and the measurement of the resulting glucose by either the glucose-oxidase/peroxidase reaction, or the use of the Trinder reagent.

### a) Hydrolysis of trehalose by trehalase.

The trehalase is obtained from the fungus *Humicola grisea var. thermoidea*. The organism is grown at 40°C on a solid medium containing 4% oat and 1.8% agar. The isolation and purification of the trehalase from this fungus is performed according to Neves et al. (FEBS Lett. 283, 19-22, 1991).

After preparation and centrifugation of the extracts according to the methods described above, 30 µl of'the extract is transferred to new microcentrifuge tubes, and 10 µl buffer (300 mM NaAc, 30 mM CaCl₂, ph 5.5) and 40 µl trehalase solution (400 U/ml) is added The mixture is incubated at 40°C for 45 min.
A trehalose standard curve of 0,1,2,4,8 and 10 mM is also analyzed.

### b) Measurement of the glucose from the hydrolysis of trehalose by trehalase by oxidation of glucose by glucose-oxidase/peroxidase.

### Materials:

Solution A: containing 3.75 mg glucose-oxidase (100 U/mg), 8 mg peroxidase (100 U/mg) and 2.25 ml Tris/Cl (1M pH8) adjusted to 100 ml with water.
Solution B: containing 10 mg/ml ortho-dianisidine-diHCl.
The glucose-oxidase solution is prepared just before use by mixing 1 ml of solution B with 100 ml solution A.
The method involves the transfer of 60 µl of the product of the trehalase reaction to a glass tube, adding I ml of the glucose-oxidase solution, incubating for 60 min at 30 °C.
The reaction is terminated by adding 0.5 ml H₂SO4 (56%) to the reaction mixture. The OD of the reaction mixture is measured at 546 nm.
A glucose standard curve with 0, 1, 2, 3 and 4 mM is analyzed at the same time.

### c). Measurement of the glucose from the hydrolysis of trehalose by trehalase by oxidation of glucose using Trinder reagent.

The glucose produced from the hydrolysis of trehalose by trehalase can also be measured using the Trinder reagent (SIGMA), which is based on the same principle as the glucose oxidase reaction.

The method involves the transfer of 20 µl of the end products of the trehalase reaction in a microtiter plate well, the addition of 200 µl Trinder reagent and mixing by shaking and incubating for 15 min at 30°C. The OD of the sample is determined at 505 nm in the microtiter plate reader (Spectramax).

A glucose standard curve with 0, 1, 2, 3 and 4 mM is analyzed at the same time.

### d) Calculation of the TPP activity.

Following the hydrolysis of trehalose by trehalase and the measurement of the resulting glucose by the glucose-oxidase/peroxidase reaction, the TPP activity can be calculated by dividing the OD of a sample by the time of the reaction and by the protein content (expressed as nKat/g protein).

Alternatively the trehalose that is formed by the action of the trehalose-6-phosphate enzyme can be measured by the HPLC analysis on a CarboPac PA-100 anion-exchange column as described by De Virgilio et al. (Eur. J. Biochem.212, 315-2-323, 1993).

### 3. Measurement of the phosphate released from the trehalose-6-phosphate.

Apart from the trehalose, the phosphate can also be measured that is released by the action of the trehalose-6-phosphate phosphatase enzyme.

This can be performed by either one of the following methods:
a) Continuous phosphate measurement using the EnzChekTM Phosphate Assay Kit (Bioprobes). The substrate 2-amino-6-mercapto-7-methylpurine riboside (MESG) with maximum absorbance at 330 nm, is converted by purine nucleoside phosphorylase (PNP) to a product with maximum absorbance at 360 nm. The increase in absorbance at 360 nm is monitored spectrophotometrically and is proportional to the phosphate consumption by the MESG/PNP reaction.
b) Free phosphate measurement by the method of Bencini (Anal. Biochem. 132: 254-258, 1983). The reagent solution, an aqueous mixture of ammonium molybdate (100 mM ) and zinc acetate (15 mM) at pH 5, produces a stable complex with orthophosphate that absorbs strongly at 350 nm. The method is linear up to 300 pM phosphate
c) The measurement of phosphate according to the method of Fiske-Subbarow comprises:
   1. the addition of 900 µl of 0.5% ammonium molybdate in 0.8N HCI to each sample;
   2. the addition of 100 µl of Fiske-Subbarow solution (0.025% 1-amino-2-naftol-4-sulfonic acid + 0.5% Na₂SO₃ + 15% Na₂S₂O5);
   3. after incubation at room temperature for 30 min, the OD is measured at 700 nm;
   4. the protein concentration is determined by the method of Lowry (Lowry et al., J Biol. Chem. 193, 265-275, 1951)
d) The measurement of free phosphate using glyceraldehyde-3-phosphate dehydrogenase and phosphoglycerate kinase in a linked enzyme assay according to Trentham et al. (Biochem. J. 126, 635-644, 1972).

### 4. Measurement of TPP activity using radioactive trehalose-6-phosphate as substrate

Alternatively TPP activity can be measured by direct measurement of radioactive phosphate using radioactive trehalose-6-phosphate in the extracts as has been described by Vuorio et al. (Eur. J. Biochem. 216, 849-861,1993). The method comprises the following steps:
a) cultures are grown to the desired density and are cooled quickly on ice;
b) preferably, at least 400 mg of cells are harvested by centrifugation for 3 min at 3000 rpm;
c) cells are washed twice with ice-cold distilled water;
d) cells are resuspended in 990 µl extraction buffer consisting of 968 µl of 50 mM Imidazole (Merck), 2 µl of 0.5 M EDTA (Tritriplex III Merck), 20 µl of 0.1 M MgCl2 (UCB),
e) cells are transferred to screw capped tubes and an equivalent of 0.5 ml glass beads with 0.5 mm diameter are added;
f) just before extraction 10 µl of a 100 mM PMSFsolution (Sigma) in methanol; is added to the cell mixture ;
g) the extraction is performed in a Fastprep apparatus (BIO101) for 20 seconds at level 4, and is repeated three times with a cooling step on ice in between.

Alternatively the extraction can be performed on a vortex apparatus in glass tubes;
h) extracts are centrifuged at 4°C for 20 min at 14000 rpm;
i) 200 µl of the supernatant is loaded on a small Sephadex G25 column, made in a blue tip containing a siliconized glass bead. The tip is filled completely with pre-equilibrated G25 Sephadex (50 mM Tricine (Sigma) buffer pH7). The tips are centrifuged once before application of the extract for 1 min at 800 rpm. The extract is centrifuged for 1 min at 1000 rpm;
j) 5 µl of the eluate is added to 45 µl assay solution consisting of 27.5 mM Tris-CI (pH7 4), of 5.5 mM MgCl₂, 1 mg/ml BSA and 0.55 mM trehalose-6-phosphate (specific activity 854 cpm/nmol);
k) the assay mixture and the control mixture are incubated for 1 hour at 30°C;
l) the assay mixture is boiled for 5 min to stop the reaction;
m) AG1-X1 is added to the mixture and the mixture is incubated for 20 min at room temperature.
n) centrifugation of the micro-centrifuge tubes for 5 min at 14000 rpm
o) after centrifugation 400 µl of the supernatant is counted in a liquid scintillation counter.

### STRAINS USED INCLUDING THE TPS2Δ MUTANT

The following strains were used in the experiments :

| Name | Relevant genotype | Complete genotype |
|---|---|---|
| W303.1A | Wild type | a *leu2-3, 112 ura3-1 tpr-1 his3-11, 15 ade2-1 can1-100 GAL SUC2* |
| YSH448 | *tps2*Δ | a *leu2-3, 112 ura3-1 tpr1-1 his3-11,15 ade2-1 can1-100 GAL SUC2 tps2*Δ*::HIS3* |
| PVD32 | W303.1A prototrophic | |
| PVD23 | *tps2*Δ prototrophic | |
| PVD45 | YEpTPS2 | *a leu2-3*/*112 ura3-1 tpr1-1 his3-11, 15 ade2-1 can1-100 GAL SUC2 tps1*Δ*::TRP1 tps2*Δ*::LEU2* + *pSAL4*/*TPS2 (URA3)* |
| YSH339 | W303.1 A alpha | alpha *leu2-3, 112 ura3-1 tpr1-1 his3-11, 15 ade2-1 can1-100 GAL SUC2* |
| PVD190 | heterozygotic W303 diploid | *leu2-3,112/LEU2 ura3-1*/*URA3 trp1-1*/*TRP1 his3-11,15*/*HIS3 ade2-1*/*ADE2 can1-100 GAL SUC2* |
| PVD191 | *tps2*Δ/WT diploid | *leu2-3,112 ura3-1*/*URA3 trp1-1*/*TRP1 his3-11,15*/*HIS3 ade2-1*/*ADE2 can1-100 GAL SUC2 tps2*Δ*::LEU2* |

The construction of the prototrophic strains was performed as follows:

### 1. Construction of PVD32.

YSH202 was first transformed with a plasmid containing the *HIS3* marker. The plasmid pJJ215 was linearized with *Nhe*1, which cuts in the marker. The complete plasmid will integrate at the location of the *his3* marker and as a result one wild type *HIS3* marker is present in the genome. The resulting strain is PVD1: W303-1 A *HIS3* Subsequently PVD1 was transformed with a plasmid containing the *URA3* marker. The plasmid YIplac 211 was linearized with *Eco*RV, which cuts the marker. The complete plasmid will integrate at the location of the *ura3* marker and as a result one wild type *URA3* marker is present in the genome. The resulting strain is PVD2: W303-1 A *HIS3 URA3*, PVD2 was transformed with a plasmid containing the *LEU2* marker. The plasmid YIplac128 was linearized with *Eco*RV, which cuts in the marker. The complete plasmid will integrate at the location of the *leu2* marker and as a result one wild type *LEU2* marker is present in the genome. The resulting strain is PVD16: W303-1A *HIS3 URA3 LEU2*. PVD6 was transformed with a plasmid containing the *ADE2* marker. The plasmid pASZ10 was linearized with *Eco*RV, which cuts in the marker. The complete plasmid will integrate at the location of the *ade2* marker and as a result one wild type *ADE2* marker is present in the genome. The resulting strain is PVD29: W303-1A *HIS3 URA3 LEU2 ADE2*. Finally, PVD29 was transformed with a plasmid containing the *TRP1* marker. The plasmid Ylplac204 was linearized with *Eco*RV, which cuts in the marker. The complete plasmid will integrate at the location of the *trp1* marker and as a result one wild type *TRP1* marker is present in the genome. The resulting strain is PVD32: W303-1A *HIS3 URA3 LEU2 ADE2 TRP1*.

### 2. Construction of PVD23

YSH448 was first transformed with a plasmid containing the *HIS3* marker. The plasmid pJJ215 was linearized with *Nhe*1, which cuts in the marker. The complete plasmid will integrate at the location of the *his3* marker, and as a result one wild type *HIS3* marker is present in the genome. The resulting strain is PVD11: *tps2*Δ::*LEU2 HIS3*. PVD11 was subsequently transformed with a plasmid containing the *URA3* marker. The plasmid Ylplac211 was linearized with *Eco*RV, which cuts in the marker. The complete plasmid will integrate at the location ofthe *ura3* marker and as a result one wild type *URA3* marker is present in the genome. The resulting strain is PVD7: *tps2*Δ::*LEU2 HIS3 URA3*, PVD7 was transformed with a plasmid containing the *TRP1* marker. The plasmid Ylplac204 was linearized with *Eco*RV, which cuts in the marker. The complete plasmid will integrate at the location of the *trp1* marker and as a result one wild type *TRP1* marker is present in the genome. The resulting strain is PVD18: *tps2*Δ::*LEU2 HIS3 URA3 TRP1*. Finally, PVD18 was transformed with a plasmid containing the *ADE2* marker. The plasmid pASZ10 was linearized with *Eco*RV, which cuts in the marker. The complete plasmid will integrate at the location of the *ade2* marker and as a result one wild type *ADE2* marker is present in the genome. The resulting strain is PVD23: *tps2*Δ::*LEU2 HIS3 URA3 TRP1 ADE2*.

### 3. Construction of PVD190.

Strain PVD190 is a diploid strain made by a cross between the W303 prototrophic strain (PVD32) and the W303 haploid auxotrophic strain (YSH339).

### 4. Construction of PVD191.

Strain PVD191 is a diploid strain made by a cross between the *tps2*Δ prototrophic strain (PVD32) and the W303 haploid auxotrophic strain (YSH339).

### EXPERIMENTAL

### Sensitivity of the tps2Δ strain towards antifungals

Sensitivity towards the antifungals itraconazole and ketoconazole was tested in microtiter wells using a bioscreen C apparatus (Life sciences, Labsystems) and on solid culture media.

Yeast cells were pre-grown in YPglucose medium till stationary phase. The cells were diluted to a initial optical density of 0.05 OD and 300 µl of cell suspension was added to each well of a microtiter plate. Three µl of the correct stock solution of the compound, dissolved in DMSO, were added to each well. For the control reactions, only DMSO was added. The microtiter plate was placed in the bioscreen C apparatus and were incubated at 33°C or 37°C, with medium intensity shaking (30 seconds shaking per minute). The optical density at 600 nm was measured every 30 min.

The experiments proved to be reproducible if the strains are pre-grown and collected at the stationary phase. During the exponential growth phase on glucose medium, no trehalose synthesis occurs and hence no trehalose-6-phosphate is accumulated and hence the antifungals have similar effects on the *tps2****Δ*** strain as on the wild type (not shown).

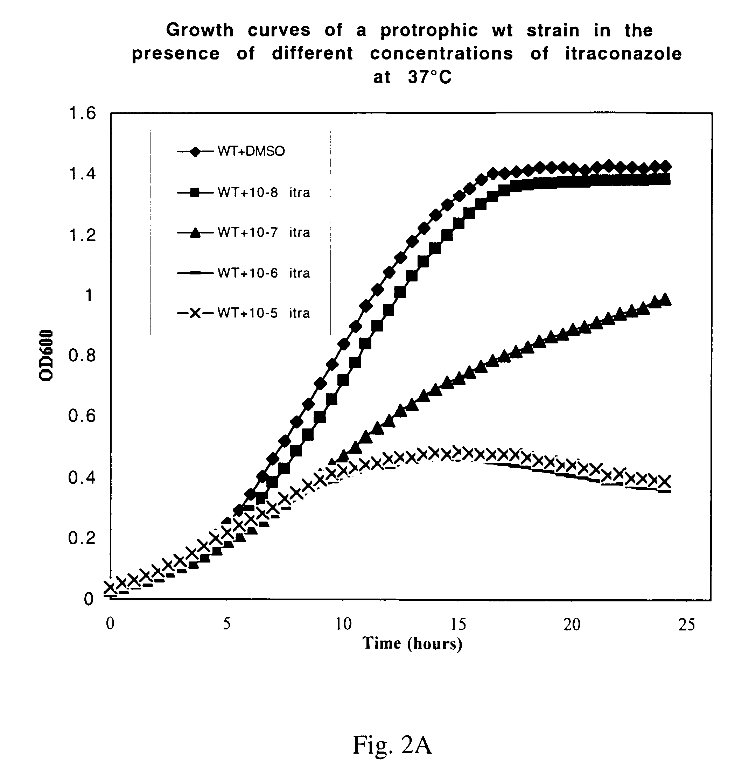

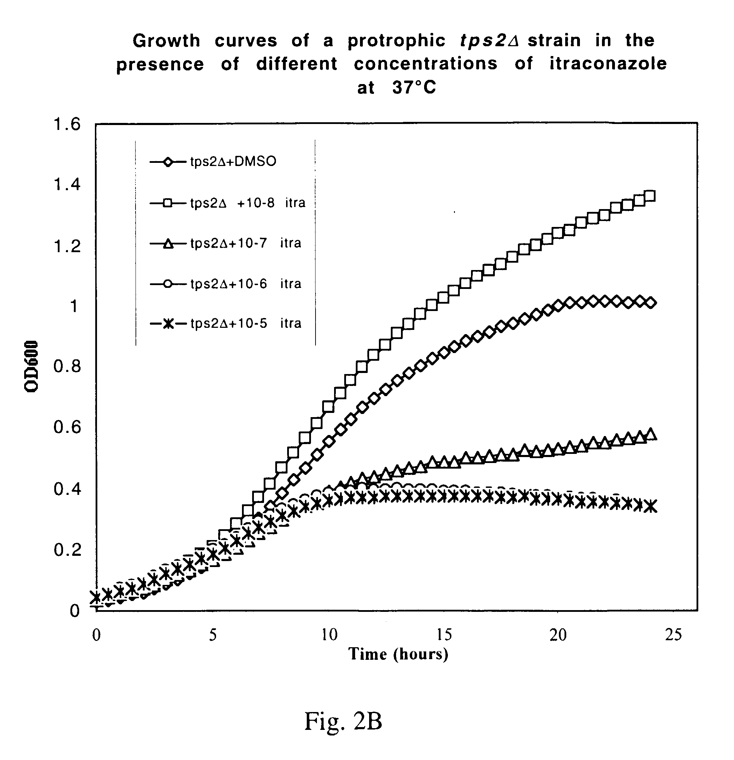

At 37°C, the growth of the *tps2*Δ mutant strain PVD23 in the presence of 10⁻⁵ to 10⁻⁸ M Itraconazole or 10⁻⁵ to 10⁻⁸ M Ketoconazole was compared to the growth of strain PVD32 and to the growth of both strains in the absence of antifungal agents, as shown in Figure 2. At this temperature the *tps2*Δ strain is already affected by itself for growth on the glucose medium (YPD + DMSO).

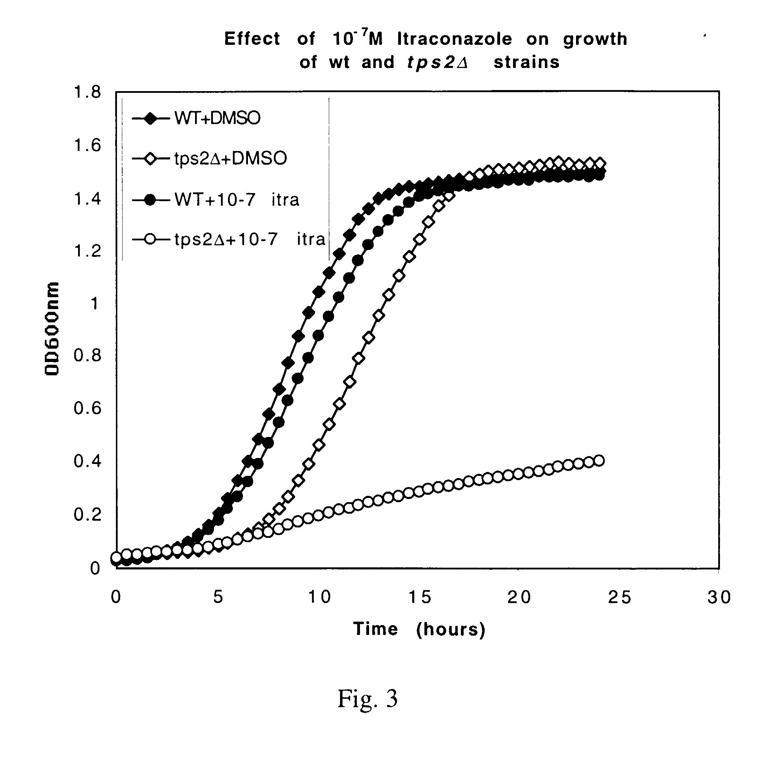

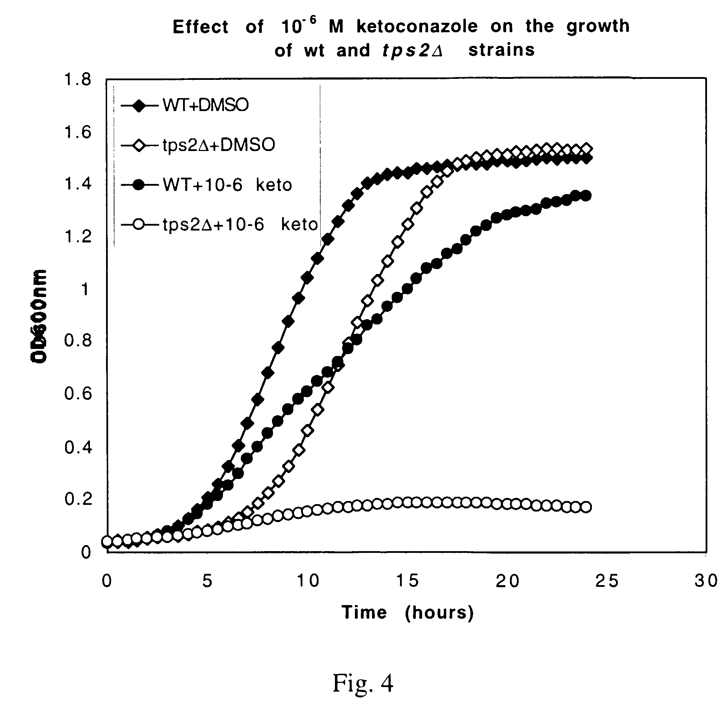

The effect of the addition of 10⁻⁷ M Itraconazole and 10⁻⁶ Ketoconazole on the growth curve of prototrophic *tps2*Δ strain PVD32 and wild type strain PVD23 at 33°C is shown in Figures 3 and 4 respectively.

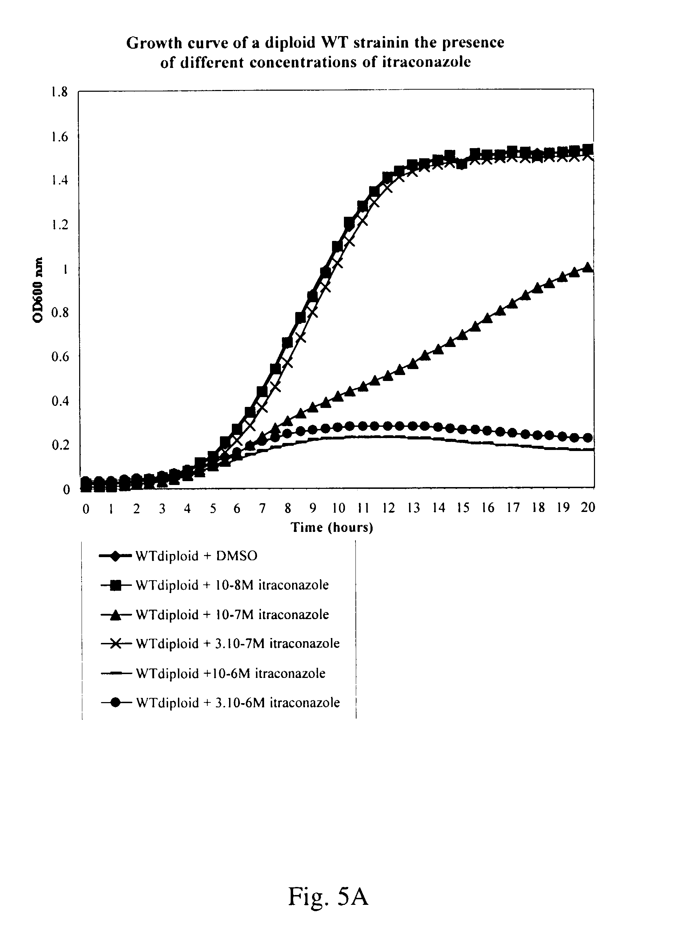

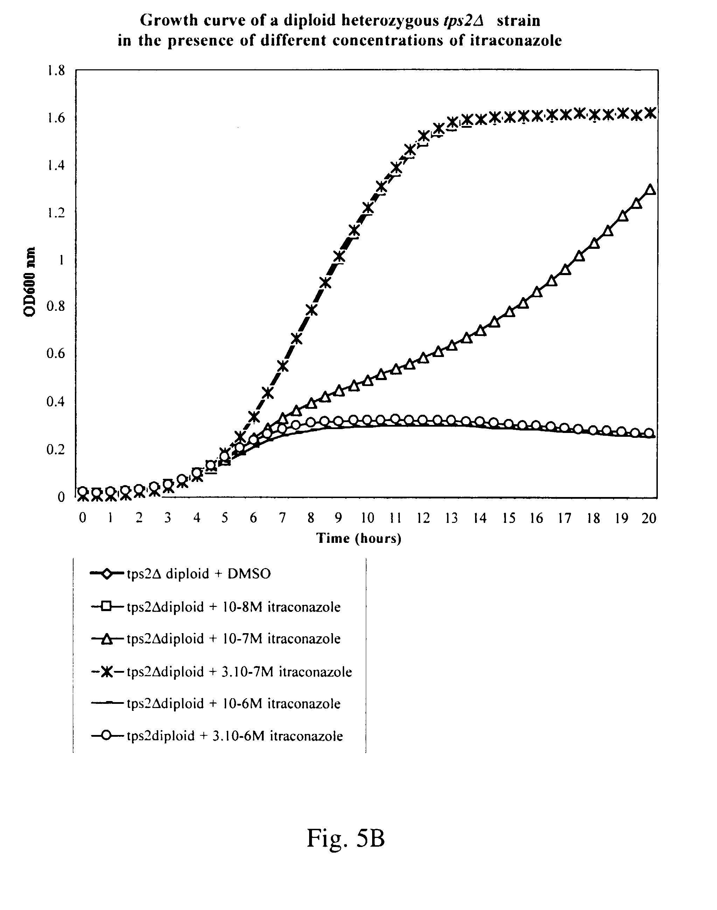

Addition of 10⁻⁷ M Itraconazole or 10⁻⁶ M ketoconazole to the *tps2*Δ strains had a dramatic effect on their growth, with no detectable growth occurring after 24 hours. The effect of different concentrations of itraconazole on the growth at 33°C of the diploid wild type strain (PVD190) and the diploid heterozygous *tps2*Δ strain PVD191 was tested and the results are presented in Figure 5.

### RESULTS

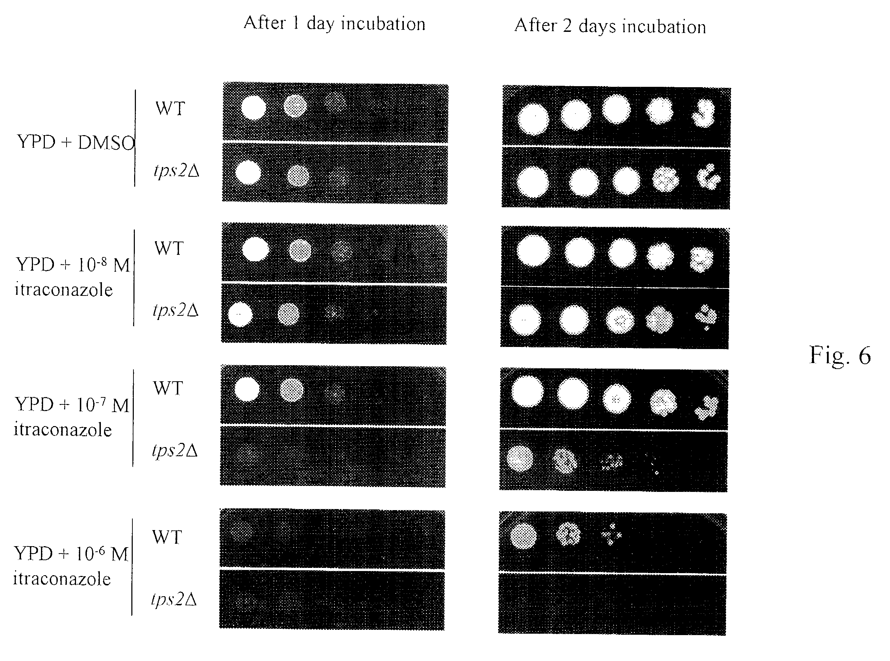

The effect of Itraconazole on the growth of wild type (PVD32) and *tps2*Δ (PVD23) strains using YPD plates containing this compound was tested and the results are presented in Figure 6. Cells were pregrown on YPD glucose medium till stationary phase. The cells were diluted to an initial OD of 0.5. This corresponds to approximately 10⁷ cells/ml. Starting from this initial cell suspension, 10-fold serial dilutions were made and from each dilution 10 µl suspension was spotted on YPD plates containing different concentrations of Itraconazole. The plates were incubated at 33°C and the occurrence of growth was estimated after 1 and 2 days.

At 33°C and 10⁻⁷ M Itraconazole incorporated in the medium, the *tps2*Δ strain was inhibited for growth, even after two days incubation, thus confirming the results obtained with the liquid media.

### The TPS2 deletion strain is sensitive to osmotic and heat stress..

The growth characteristics of wild type (PVD32) and *tps2*Δ (PVD23) strains were tested in the presence of either 1.5 M sorbitol or 5% NaCl. The strains were pre-grown on YPD plates to stationary phase. The cells were diluted to an OD of 0.5. This corresponds to approximately 10⁷ cells/ml Starting from this initial cell suspension, 10-fold serial dilutions were made and from each dilution 10 µl suspension was spotted on YPD plates containing either 1.5 M sorbitol or 5% NaCl. Plates of the different media were inoculated with 5 µl of 10-fold serial dilutions and incubated at 30 °C.

The effect of heat stress on the prototrophic wild type and *tps2*Δ strains was tested by inoculating YPD plates with 10 µl of cell suspension and incubating the plates at 37°C, 39°C and 41°C. Visual readings were done after 24 and 48 hours.

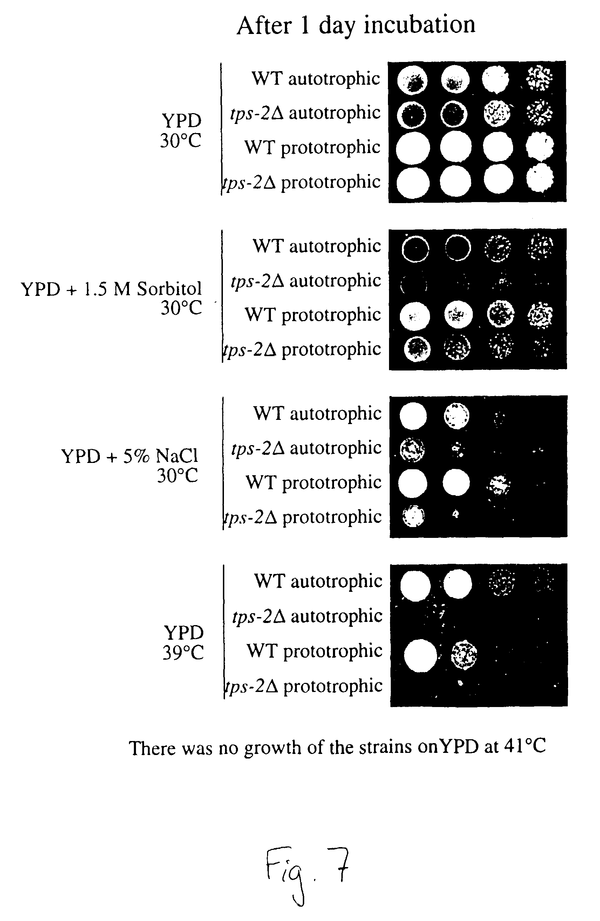

Figure 7 shows the effect of the osmotic and heat stress on the growth of the tested strains. The results clearly show that a *tps2*Δ strain is more sensitive to osmotic or salt stress in comparison with a wild type strain.

A heat stress experiment has been repeated where the yeast cells were incubated at 39°C A *tps2*Δ strain cannot grow at this temperature, whereas a wild type strain grows perfectly well. Remarkable was the fact that the *tps2*Δ strain in the W303.1A background did not show any growth defect on plates at 37°C. In liquid media, however, there is a difference in growth rate between the wild type and the mutant. At 41°C, none of the strains grew.

### SCREENING ASSAY

Several known inhibitors of different phosphatase enzymes have been tested, such as vanadate, tetramisole, (-)-p-bromotetramisole, (+)-p-bromotetramisole, levamisole, N-ethylmaleimide (NEM) and Dithiodinitrobenzoate (DTNB) for their inhibitory effect on TPP activity

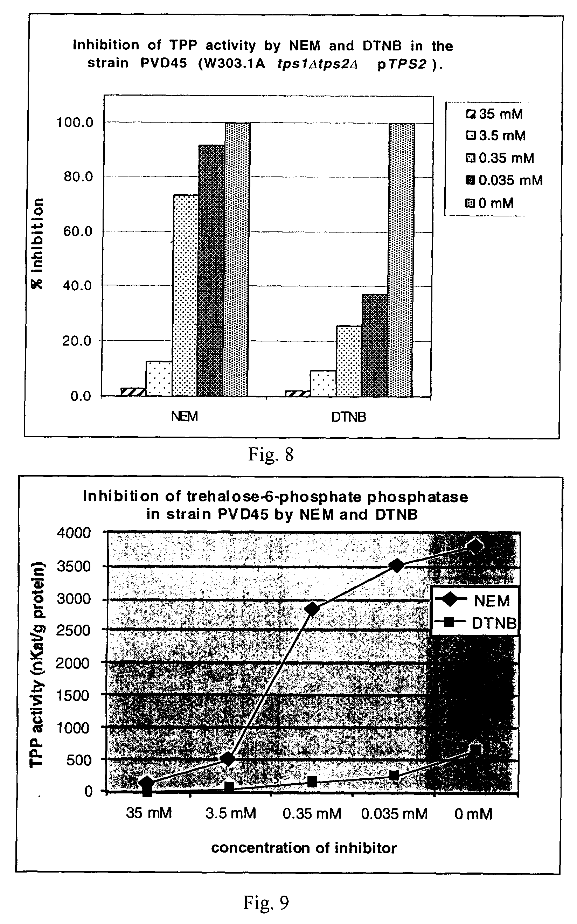

To perform a screening assay with the different candidate inhibitory compounds, strain PVD45 (PVD45: a *leu2-3/112ura3-1 trp1-1 his3-11*/*15 ade2-1 can1-100GAL SUC2 tps1*Δ*::TRP1 tps2*Δ*::LEU2 + pSAL4*/*TP2S (URA3))* was used which over-expresses *TPS2* from *Saccharomyces cerevisiae.*
The screening assay comprises the following steps:
1. Preparation of the extracts.
   The strain PVD45 was grown in 50 ml SDgal-ura medium to stationary phase. Extracts were prepared according to the protocol described previously on page 10.
2. Screening of the candidate inhibitory compounds NEM and DTNB.
   Stock solutions of 100 mM N-ethylmaleimide (NEM) and 100 mM Dithiodinitrobenzoate (DTNB) are prepared in water and ethanol respectively.
   From these stock solutions, serial dilutions of 0.1 mM, 1mM and 10 mM, are made. For the experiments 56 µl of each dilution of the compound is added to the Assay I solution consisting of 40 µl of 200 mM Tricine buffer (pH7), 20 µl of 0.1 M MgCl₂, 20 µl trehalose-6-phosphate (Sigma) in 4 µl H₂O. For the Assay I control mixture 20 µl trehalose-6-phosphate is omitted and replaced by 20 µl H₂O. Thus in the experiments the following final concentrations of the tested compounds NEM and DTNB were used: 35 mM, 3.5 mM, 0.35 mM, 35 µM and 0 µM (= control).
   To the assay mixture 20 µl of extract is added.
   The assay mixtures and the control mixtures are incubated for 30 min at 30°C; subsequently boiled for 5 min to stop the reaction and cooled down to room temperature. The micro-centrifuge tubes were centrifuged for 5 min at 14000 rpm.
   Measurement of the trehalose-6-phosphate activity is performed according to the methods described previously.
The marked inhibition of trehalose-6-phosphate phosphatase activity in strain PVD45 by NEM and DTNB is shown in Figures 8 and 9. None of the following compounds inhibited TPP activity: vanadate, tetramisole, (-)-p-bromotetramisole, (+)-p-bromotetramisole, levamisole. The above mentioned selective and specific inhibitory action of NEM and DTNB whereas general phosphatase inhibitors had no inhibitory effect on trehalose-6-phosphate phosphatase indicates that specific inhibitors can be found for TPP activity which will not interfere with phosphatase enzymes of host cells.

An assay in accordance with the present invention also involves screening test inhibitory compounds from large libraries of synthetic or natural compounds. Synthetic compound libraries are commercially available from, for example, Maybridge Chemical Co. (Trevillet, Cornwall, UK), Comgenex (Princeton, NJ), Brandon Associates (Merrimack, NH), and Microsource (New Milford, CT). A rare chemical library is available from Aldrich Chemical Company, Inc. (Milwaukee, WI). Alternatively, libraries of natural compounds in the form of bacterial, fungal, plant and animal extracts are available from, for example, New Chemical Entities, Pan Laboratories, Bothell, WA or MycoSearch (NC), Chiron, or are readily producible. Plant extracts may also be obtained from the University of Ghent, Belgium. Additionally, natural and synthetically produced libraries and compounds are readily modified through conventional chemical, physical, and biochemical means.

The present invention further includes a method for treating fungal infection in a patient in need of such treatment, comprising administering to said patient an antifungal agent identified according to the above assay method or a pharmaceutically acceptable salt, ester or pro-drug thereof.

More generally, the invention includes a method for treating a fungal, a bacterial infection, or a nematode or insect infestation, in an animal or a plant in need of such treatment which comprises administering a specific inhibitor which inhibits a fungal enzyme converting with a low or high degree of specificity a sugar phosphate into a sugar or a sugar alcohol phosphate into a sugar alcohol that are accumulated in large quantities by cells for instance, but not exclusively, under conditions deviating from the optimal growth condition or as a reaction to stress conditions, the inhibition being either directly of the enzyme or indirectly, e.g. by suppressing the expression of the corresponding gene. The inhibitor may be determined by the asssays described above.

Compositions in accordance with the present invention include a biologically or therapeutically effective amount of an inhibitory agent (either biocide or pharmaceutucal) determined in accordance with a screening assay in accordance with the present invention. Therapeutically or biologically effective amounts are those quantities of the active agent of the present invention that afford prophylactic protection against the relevant infections or infestations in plants or animals, and which result in amelioration or cure of an existing infection or infestation in plants or animals.

The biologically or therapeutically active agents or compositions can be formed into dosage unit forms, such as for example, creams, ointments, lotions, powders, liquids, tablets, capsules, suppositories, sprays, or the like. If the antifungal composition is formulated into a dosage unit form, the dosage unit form may contain an antifungal effective amount of active agent.

The active agents and compositions of the present invention are useful for preventing or treating fungal infections in plants and animals Fungal infection prevention methods incorporate a prophylactically effective amount of an antifungal agent or composition. A prophylactically effective amount is an amount effective to prevent fungal infection and will depend upon the fungus, the agent and the host. These amounts can be determined experimentally by methods known in the art. Fungal infection treatment methods incorporate a therapeutically effective amount of an antifungal agent or composition. A therapeutically effective amount is an amount sufficient to stabilize or to ameliorate a fungal infection.

The prophylactically and/or therapeutically effective amounts can be administered in one administration or over repeated administrations. Therapeutic administration can be followed by prophylactic administration, once the initial fungal infection has been resolved.

The antifungal agents and compositions can be applied to plants topically or non-topically, i.e., systemically. Topical application is preferably by spraying onto the plant. Systemic administration is preferably by application to the soil and subsequent absorption by the roots of the plant.

The antifungal agents and compositions can be administered to animals topically or systemically.

## Claims

1. A test method for assessing the activity of candidate substances as inhibitors of a first cell enzyme converting a sugar phosphate into a sugar or a sugar alcohol phosphate into a sugar alcohol in cells, the sugar or sugar alcohol being accumulated in large quantities by the cells for instance, but not exclusively, under conditions deviating from the optimal growth condition of the cells or as a reaction to stress conditions, the inhibition being either directly of the first enzyme or indirectly; the method comprising the steps of:
Step 1: contacting a candidate inhibitor with a biological medium comprising the sugar phosphate or sugar alcohol phosphate and the first enzyme;
Step 2: measuring activity in the medium which depends upon the activity of the first enzyme;
Step 3: repeating steps one and two with further candidate inhibitors; and
Step 4: selecting at least one candidate inhibitor which reduces activity of the enzyme compared with the same medium without the inhibitor under the same conditions.

2. The method according to claim 1, wherein the first cell enzyme is a phosphatase which synthesizes a sugar or sugar alcohol as a reaction to stress.

3. The method according to claim 1 or 2, wherein the reduction in activity is preferably at least 25%, more preferably at least 50%, more preferably at least 75%, more preferably at least 85% and most preferably at least 95%.

4. The method according to any previous claim, further comprising the steps of:
step 5: assessing the activity of a second cell enzyme which is involved in the synthesis of the corresponding sugar phosphate or sugar alcohol phosphate; and the selecting step includes selection of inhibitors which reduce the activity of the first enzyme while maintaining a viable activity of the second enzyme.

5. The method according to claim 4, wherein a viable activity of the second enzyme is at least 25%, more preferably at least 50% and most preferably at least 75% of the activity of the second enzyme in the same medium under the same conditions but without the inhibitor.

6. The method according to any previous claim, wherein the medium includes sub-cellular organelles or sub-cellular non-organelle components, a cell culture, or animal tissue, or an animal.

7. The method according to claim 6, wherein the sub-cellular organelles or sub-cellular non-organelle components or the cell culture are obtained from cells from an insect or a nematode or a fungus or a bacterium.

8. The method according to claim 6, wherein the medium is a cell culture and the first enzyme is an intracellular enzyme.

9. The method according to any previous claim wherein the first enzyme is an enzyme controlling a metabolic pathway which has an intermediary compound which is normally produced as a reaction to stress conditions and which is toxic to the cell.

10. The method according to claim 9, wherein the first enzyme is one of trehalose-6-phosphatase, glycerol-3-phosphatase, mannitol-1-phosphatase, sorbitol-6-phosphatase, arabitol-5-phosphatase, and erythritol-4-phosphatase.

11. The method according to any previous claim, wherein steps 1 is carried out with the first enzyme *in vitro*, further comprising the steps after step 4 of:
contacting the candidate inhibitors selected in step 4 with a biological medium comprising whole cells having the first enzyme as an intracellular enzyme; and
selecting those candidate inhibitors which reduce the growth of the cells.

12. The method according to any previous claim, further comprising the step of:
using a selected inhibitor in a pharmaceutical preparation or a biocide.

13. An inhibitor obtained by the method of any of the claims 1 to 11.

14. An inhibitor obtainable by the method of any of the claims 1 to 11, the inhibitor inhibiting in cells a cell enzyme converting a sugar phosphate into a sugar or a sugar alcohol phosphate into a sugar alcohol, the sugar or sugar alcohol being accumulated in large quantities by the cells for instance, but not exclusively, under conditions deviating from the optimal growth condition of the cells or as a reaction to stress conditions, the inhibition being either directly of the first enzyme or indirectly.

15. A pharmaceutical preparation including the inhibitor of claim 13 or 14.

16. The pharmaceutical preparation according to claim 15, further comprising an antifungal drug.

17. The pharmaceutical preparation of claim 16, wherein the antifungal drug is one of Amphotericin B, Flucytosine, Ketoconazole, Miconazole, Fluconazole, and Itraconazole.

18. A biocide acting on fungi, insects, nematodes, bacteria or other organisms accumulating large quantities of a sugar alcohol or a sugar in response to stress comprising the inhibitor of claim 13 or 14.

19. The biocide of claim 18, further comprising an antifungal agent.

20. The biocide of claim 19 wherein the biocide is an azole.
